(19) Europäisches Patentamt
European Patent Office
Office européen des brevets

(11) EP 0 398 774 B1

(12) **FASCICULE DE BREVET EUROPEEN**

(45) Date de publication et mention de la délivrance du brevet:
**10.09.1997 Bulletin 1997/37**

(51) Int. Cl.[6]: **C07B 39/00**

(21) Numéro de dépôt: **90401085.7**

(22) Date de dépôt: **23.04.1990**

# (54) Procédé de préparation de dérivés bromés notamment à partir d'alcools

Verfahren zur Herstellung von bromierten Derivaten aus Alkoholen

Process for the preparation of brominated derivatives from alcohols

(84) Etats contractants désignés:
**AT BE CH DE DK ES FR GB GR IT LI LU NL SE**

(30) Priorité: **02.05.1989 FR 8905811**

(43) Date de publication de la demande:
**22.11.1990 Bulletin 1990/47**

(73) Titulaire: **RHONE-POULENC CHIMIE**
**92408 Courbevoie Cédex (FR)**

(72) Inventeurs:
- **Mas, Jean-Manuel**
  **F-69100 Villeurbanne (FR)**
- **Metivier, Pascal**
  **F-69001 Lyon (FR)**

(74) Mandataire: **Ricalens, François et al**
**RHONE-POULENC CHIMIE,**
**Direction de la Propriéte Industrielle,**
**25, quai Paul Doumer**
**92408 Courbevoie Cédex (FR)**

(56) Documents cités:
**FR-A- 2 156 566**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 398 774 B1

## Description

La présente invention concerne la préparation de dérivés bromés et plus particulièrement la préparation de ces dérivés à partir d'alcools.

Les dérivés bromés sont des produits intéressants industriellement notamment comme intermédiaires de synthèse, agents de protection des plantes ou insecticides.

On connaît de nombreux procédés de bromation d'alcools utilisant des réactifs bromant variés tels que l'acide bromhydrique, $SOBr_2$, $PBr_3$, l'association triphenylphosphine/brome par exemple.

Mais il s'agit souvent de procédés non industriels à rendement médiocre ou utilisant des réactifs coûteux ou dangereux tel que $SO_{Br2}$.

Dans le cas particulier de la synthèse du bromométhyl-2 tétrahydropyranne, produit utilisable comme nématicide par exemple, l'emploi de l'acide bromhydrique ne donne que de mauvais rendements à cause de la fragilité du cycle en milieu acide. On est amené à utiliser alors comme réactif le dibromure de triphenylphosphine mais, compte-tenu du prix de celui-ci, il s'agit là d'un procédé peu industriel.

Un premier objet de l'invention est donc un procédé qui soit peu coûteux et de rendement important en dérivés bromés.

Un second objet de l'invention est un procédé remplissant les mêmes conditions pour la bromation d'alcools.

Un troisième objet de l'invention est un procédé du type ci-dessus qui soit stéréosélectif.

Dans ce but le procédé, selon l'invention, de préparation d'un dérivé bromé est caractérisé en ce qu'il comprend au moins une étape dans laquelle on transforme un réactif chloré en un réactif bromé, par action d'un agent bromant. En particulier selon une mise en oeuvre de l'invention, on fait réagir avec un agent bromant le chloroformiate, le chlorosulfite ou le chlorophosphite correspondant au dérivé bromé précité.

Selon une variante particulière le procédé de l'invention s'applique à la bromation d'un alcool et il est caractérisé en ce qu'il comprend les étapes suivantes :

- on met en contact l'alcool avec au moins un réactif choisi parmi le chlorure de thionyle, le phosgène, l'oxychlorure de phosphore ;
- on met en contact le milieu réactionnel ou le produit de réaction issu de l'étape précédente avec un agent bromant.

Selon une autre variante de l'invention, le procédé de bromation d'un alcool est caractérisé en ce qu'on fait tout d'abord réagir un agent bromant avec un réactif choisi parmi le chlorure de thionyle, le phosgène et l'oxychlorure de phosphore puis ensuite on met en contact le milieu réactionnel ou le produit de réaction ainsi obtenu avec l'alcool à bromer.

Grâce au procédé de l'invention, on arrive à des rendements qui peuvent atteindre plus de 90 % dans certains cas et ceci avec des réactifs industriels.

D'autres détails et caractéristiques de l'invention apparaîtront plus clairement à la lecture de la description et des exemples non limitatifs qui vont suivre.

Une caractéristique du procédé de l'invention consiste à obtenir le dérivé bromé en partant de ou en passant, au cours de la synthèse, par le chloroformiate, le chlorosulfite ou le chlorophosphite correspondant à ce même dérivé bromé. Ici et pour le reste de la description, le terme chlorophosphite couvre le monochlorophosphite et le dichlorophosphite.

Dans le cas de la variante de l'invention où l'on applique le procédé à la bromation d'un alcool, on part alors de l'alcool correspondant au dérivé bromé que l'on cherche à obtenir et on prépare le chloroformiate, le chlorosulfite ou le chlorophosphite précité par mise en contact de l'alcool avec respectivement le phosgène, le chlorure de thionyle ou l'oxychlorure de phosphore.

Une fois cette étape préliminaire achevée et donc une fois obtenu le dérivé chloroformiate, chlorosulfite ou chlorophosphite, dans un second temps, on peut mettre en contact ou faire réagir le produit obtenu avec un agent bromant de la manière décrite plus haut. Il est donc entendu pour le reste de la description que l'ensemble des caractéristiques décrites pour les étapes ultérieures s'appliquent intégralement et identiquement aux deux variantes mentionnées ci-dessus.

Le procédé de l'invention s'applique à la préparation d'un large type de dérivés bromés, à partir notamment des alcools primaires ou secondaires. Plus particulièrement on peut partir des alcools de formule (1).

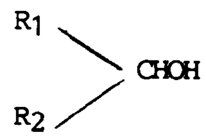
(1)

dans laquelle $R_1$ et $R_2$ peuvent être identiques ou différents et représentent l'hydrogène un radical alkyle, alcényle, alcynile, ces radicaux pouvant être linéaires ou ramifiés, un radical cycloalkyle, alkylcycloalkyle, terpénique, $CO_2R_3$, -$(CH_2)_n$ $CO_2R_3$, -$COR_3$, -$SOR_3$, -$SO_2R_3$, n étant un nombre entier, $R_3$ étant l'hydrogène ou un radical alkyle, alcényle, alcynile, ces radicaux pouvant être linéaires ou ramifiés, cycloalkyle, alkylcycloalkyle, terpénique, aryle notamment aryle substitué; au moins un de $R_1$ et $R_2$ pouvant en outre former un hétérocycle comportant en remplacement d'un ou plusieurs atomes de carbone un ou plusieurs hétéroatomes choisis parmi l'oxygène, le soufre ou l'azote.

Généralement n varie entre 1 et 8 de préférence entre 1 et 4.

On obtient notamment ainsi par le procédé de l'invention les produits bromés de formule (2)

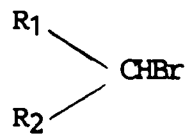
(2)

dans laquelle $R_1$ et $R_2$ sont tels que définis pour la formule (1).

On peut aussi partir des chloroformiates, chlorosulfites et chlorophosphites correspondants, c'est-à-dire des produits de formules (3) (4) et (5) ou (6) respectivement

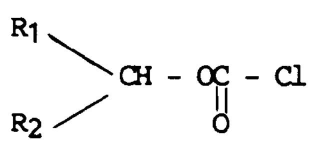
(3)

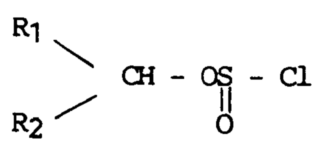
(4)

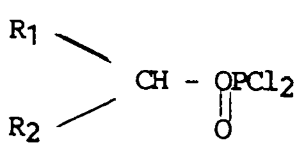
(5)

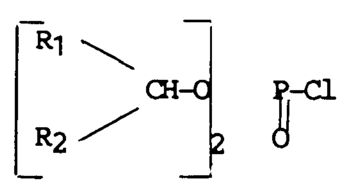
(6)

$R_1$ et $R_2$ étant tels que définis précédemment.

Comme type de produits plus particuliers utilisables dans le cadre de la présente invention on peut mentionner les familles ci-dessous.

On peut ainsi partir des alcools aliphatiques saturés plus particulièrement les alcools primaires et secondaires.

A titre d'exemple on peut citer le butanol-1, l'octanol-1, le pentanol-2, l'octanol-2.

On peut aussi mettre en oeuvre des alcools éthyléniques primaires ou secondaires notamment les alcools allyliques.

On peut de même utiliser le procédé de l'invention aux acides-alcools et notamment aux acides lactiques ou aux lactates.

Comme on l'a vu plus haut, l'invention s'applique aussi aux produits comportant un hétérocycle, cet hétérocycle contenant un ou plusieurs hétéroatomes choisis parmi l'oxygène, le soufre ou l'azote.

C'est ainsi que l'on peut partir de produits de formule (1) dans laquelle au moins un de $R_1$ et $R_2$ est un hétérocycle du type furanne ou pyranne tels que par exemple

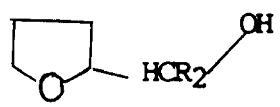

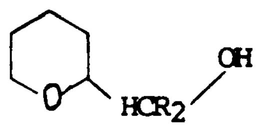

$R_2$ pouvant être notamment l'hydrogène.

On citera tout particulièrement l'hydroxyméthyl-2 tétrahydropyranne ainsi que l'hydroxyméthyl-2 tetrahydrofuranne.

Bien entendu tout ce qui a été dit ici au sujet des alcools de départ est applicable à tous les produits dérivés correspondants ou analogues de formules (3), (4), (5) et (6).

Il est important de noter que le procédé de l'invention est particulièrement avantageux dans la mesure où il permet de conduire à des produits optiquement actifs.

En effet, généralement le procédé conserve l'activité optique du produit de départ lors de la réaction d'échange hydroxyle brome.

Les différentes étapes du procédé vont maintenant être étudiées plus précisément.

Comme cela a été vu plus haut, l'invention consiste à partir des dérivés de formule (3), (4), (5) et (6). Ces dérivés peuvent avoir été préparés préalablement ou bien ils peuvent être fabriqués lors de la mise en oeuvre du procédé lui-même et c'est le cas lorsqu'on part de l'alcool correspondant.

Dans ce dernier cas, la première partie du procédé consiste à mettre l'alcool en présence de phosgène, de chlorure de thionyle ou d'oxychlorure de phosphore suivant que l'on veut obtenir les dérivés de formules (3), (4), (5) ou (6) respectivement.

Pour la mise en oeuvre de cette première partie du procédé, l'ordre d'introduction des réactifs peut être quelconque.

Par ailleurs, la réaction peut être effectuée en masse ou dans un solvant.

Dans ce dernier cas, on utilise de préférence un solvant aprotique. On peut citer notamment les hydrocarbures saturés (n pentane, 2-méthylhexane, cyclohexane), les hydrocarbures aromatiques (benzène, toluène, éthylbenzène), les cétones aliphatiques saturées ou aromatiques, les hydrocarbures halogénés saturés aliphatiques ou aromatiques, les esters aliphatiques saturés ou aromatiques, seuls ou en mélange.

On préfère généralement les hydrocarbures halogénés aromatiques ou non.

En général, on conduit la réaction à une température comprise entre 0°C et celle de reflux du solvant le cas échéant et avantageusement entre 5 et 60°C.

Si la réaction s'effectue en présence d'un solvant la dilution du produit réactionnel sera comprise entre 0,5 % et 99 % en poids par rapport au poids total de la solution et de préférence entre 5 et 50 %.

Le nombre d'équivalents de $SOCl_2$ ou $COCl_2$ est habituellement compris entre 0,9 et 3 et de préférence entre 0,9 et 1,1. Le nombre d'équivalents de $POCl_3$ sera fonction de l'intermédiaire de formule (5) ou (6) que l'on cherche à préparer. Il est généralement compris entre 0,4 et 3 de préférence entre 0,4 et 1,1.

La seconde partie du procédé dans le cas où l'on est parti d'un alcool ou la partie essentielle si l'on part directement des dérivés de formules (3), (4), (5) ou (6) consiste à faire réagir ou mettre en contact le milieu réactionnel ou le produit de réaction issu de la première partie ou les dérivés précités avec un agent bromant.

Comme agent bromant on utilise plus particulièrement un bromure acide ou un bromure d'alkylammonium.

Par bromure acide, on entend ici tout composé à base de brome susceptible de donner des ions $Br^-$ dans le milieu réactionnel.

A titre d'exemple, on peut citer l'acide bromhydrique, les bromures de métaux alcalins, tels que LiBr, NaBr, les bromhydrates d'amines tertiaires aliphatiques ou aromatiques tels que le bromhydrate de triéthylamine, de pyridine de picoline.

En ce qui concerne les bromures d'alkylammonium, on peut mentionner le bromure de tétrabutylammonium.

En ce qui concerne les conditions de la réaction (réaction en masse ou dans un solvant, température, concentration, etc ...) tout ce qui a été dit plus haut pour la première partie de la réaction s'applique aussi ici. On précisera que le nombre d'équivalents d'agent bromant est habituellement situé dans la fourchette décrite plus haut de 0,9 à 3.

A l'issue du traitement avec l'agent bromant, on obtient le dérivé bromé recherché que l'on peut recueillir ou séparer du milieu réactionnel par tout moyen convenable connu.

Toutefois, l'invention comporte plusieurs autres variantes utilisables pour augmenter le rendement en produit bromé, notamment dans le cas où les produits de départ sont moins réactifs que d'autres.

Ces variantes vont être décrites ci-dessous.

Selon une première variante, on chauffe le milieu réactionnel ou le produit de réaction issu de l'étape précédente de bromation.

Le chauffage se fait à une température généralement d'au moins 30°C plus particulièrement d'au moins 50°C, cette température pouvant monter jusqu'à celle du reflux du solvant le cas échéant.

Selon une seconde variante, on met en contact le milieu ou le produit précité issu de l'étape de bromation avec un composé comprenant de l'azote trisubstitué.

Comme composé ou réactif de ce type, on peut utiliser une amine choisie dans le groupe des amines tertiaires aliphatiques et aromatiques ou dans le groupe de la pyridine telles que par exemple les trialkylamines comme la triéthylamine, la pyridine, la picoline.

Pour ce même composé, on peut utiliser aussi les amides ou formamides comme par exemple le diméthylformamide, le diméthylacétamide.

On peut de même employer des dérivés de l'urée, tels que les urées substituées par des groupes alkyles par exemple la tétraméthylurée.

Comme précédemment, la réaction peut être conduite en masse ou en milieu solvant avec le même type de solvant que précédemment indiqué.

Habituellement, on utilise le composé comprenant de l'azote trisubstitué en quantité catalytique. Dans ce cas, le nombre d'équivalent de ce composé est compris entre $0{,}1.10^{-2}$ et 1 plus particulièrement entre 0,01 et 0,1.

Il est aussi possible de réaliser cette phase de contact avec ce composé à azote trisubstitué à chaud c'est-à-dire à une température comprise entre 30°C et le reflux du solvant.

A la fin de cette dernière étape le produit peut être séparé et recueilli par tout moyen connu.

D'autres variantes de l'invention peuvent encore être envisagées. C'est ainsi que dans le cas de la mise en oeuvre d'un composé à azote trisubstitué, il est possible d'inverser les étapes précitées, c'est-à-dire de mettre en contact tout d'abord le chloroformiate, chlorosulfite ou chlorophosphite avec le composé à azote trisubstitué puis ensuite avec l'agent bromant le milieu réactionnel ou le produit de réaction obtenu.

Il est à noter que les deux étapes peuvent même être combinées en une seule, c'est-à-dire que l'on introduit simultanément ou immédiatement l'un à la suite de l'autre le composé à azote trisubstitué et l'agent bromant.

Tout ce qui a été dit plus haut au sujet des conditions opératoires pour chacune des étapes (présence ou non d'un solvant, température, concentration,...) s'applique aussi ici pour ces deux dernières variantes.

On notera toutefois que dans le cas de la dernière variante décrite (étapes combinées), on peut utiliser de préférence les bromhydrates qui jouent à la fois le rôle de l'agent bromant et du composé à azote trisubstitué, les bromhydrates étant dans ce cas utilisés en quantités stoechiométriques.

Enfin, une dernière variante de l'invention consiste comme cela a été vu plus haut à faire réagir l'agent bromant avec un réactif choisi parmi le chlorure de thionyle, le phosgène et l'oxychlorure de phosphore.

Ensuite, dans un deuxième temps on met en contact le produit de réaction ainsi obtenu avec l'alcool à bromer, par exemple en coulant l'alcool dans le milieu réactionnel obtenu à l'étape précédente.

Il est à noter que cette variante s'applique plus particulièrement au cas du chlorure de thionyle.

Par ailleurs, on peut à l'issue de l'étape de réaction avec l'alcool mettre en oeuvre les différents autres modes de réalisation de l'invention qui ont été décrits plus haut. C'est-à-dire qu'il est possible de chauffer le produit ou le milieu réactionnel obtenu ou de le faire réagir avec un composé à azote trisubstitué avec ou sans chauffage.

Dans les différentes variantes mises en oeuvre selon l'invention, on passe par un intermédiaire plus ou moins labile, à savoir les composés obtenus par substitution du chlore par le brome pour les chloroformiates, chlorosulfites et chlorophosphites. Ce composé intermédiaire peut être utilisé in situ ou isolé.

Lorsque le composé est labile, il convient de choisir les solvants à bas poids de fusion et de travailler à des températures en dessous du point de décomposition, par exemple en dessous de 0°C.

Ces bromoformiates, bromosulfites ou bromophosphites peuvent donner par décomposition des bromures ; ainsi qu'indiqué ci-dessus, cette décomposition peut être favorisé par l'élévation de température ou l'addition d'amines inertes vis-à-vis de ces réactifs. Dans ce cas, la pyridine est considérée comme une amine, ainsi que les amides et les dérivés de l'urée.

Ces bromoformiates, bromosulfites et bromophosphites peuvent également être utilisées pour les réactions classiques qu'ils donnent avec différents réactifs. Ils peuvent être utilisés dans leur mélange réactionnel ou après isolement.

L'ensemble des indications données plus haut sur les réactifs et les conditions opératoires s'applique à chacune des étapes de cette dernière variante.

Des exemples concrets de l'invention vont maintenant être donnés.

Exemple 1

Cet exemple concerne la préparation du bromométhyl-1 tétrahydropyranne.

Dans un tétracol de 250 ml muni d'un réfrigérant ascendant surmonté d'une tête à hydrogéner, relié à un bulleur et à un piège à HCl, d'une ampoule à couler de 25 ml, d'un thermomètre et d'une arrivée de gaz reliée à une bouteille d'HBr (piège avec acide sulfurique et fiole de rétention) on charge 20,2 g (0,165 mole) de chlorure de thionyle.

On additionne alors 17,4 g (0,15 mole) d'hydroxyméthyl-2 tétrahydropyranne en 1 h 30 en maintenant une température d'environ 20-25°C dans la masse réactionnelle. Le dégagement d'HCl qui débute dès l'addition de l'alcool s'arrête environ 20 minutes après la fin de la coulée. On obtient alors une solution orange.

Puis on introduit dans la masse réactionnelle 13,35 g (0,165 mole) de HBr gaz à une température comprise entre 25 et 30°C en 1 h 30. Un nouveau dégagement d'HCl a lieu.

On additionne alors rapidement 1,5 g (0,015 mole) de triéthylamine et on chauffe le milieu réactionnel à une température de 70°C. Au cours des trois premières heures de chauffage on observe un dégagement lent de $SO_2$.

Après deux heures supplémentaires de chauffage on refroidit la masse réactionnelle. Après dilution au chlorure de méthylène le milieu est lavé jusqu'à neutralité avec de l'eau et une solution saturée en bicarbonate de sodium, puis séché et concentré. On obtient ainsi 25,9 g d'une huile visqueuse noire.

Après dégoudronnage par distillation (75-79°C sous 17 mm Hg) on obtient 21,5 g d'un liquide incolore titrant 94 % en bromométhyl-2 tétrahydropyranne (Rendement = 75,3 %).

Exemple 2

Cet exemple concerne la préparation du S(-) bromopropionate de méthyle.

Dans un réacteur de 100 ml équipé de la même manière que le tétracol de l'exemple 1 on charge 15 g (0,126 mole) de chlorure de thionyle et 25 ml de chlorure de méthylène.

On additionne alors en une heure et à 25°C 10,9 g (0,105 mole) de D-lactate de méthyle ( D/(L + D) = 96,9 % ) et on observe un dégagement de HCl. On introduit ensuite 9,6 g (0,12 mole) de HBr gaz en une heure et à température ambiante. Un nouveau dégagement de HCl a lieu.

On introduit ensuite 85 µl de pyridine (0,001 mole) et on chauffe le milieu au reflux pendant 8 heures 40.

Après refroidissement à température ambiante du milieu réactionnel on dose par chromatographie en phase gazeuse (Etalon dichlorobenzène) 77,4 % de bromopropionate de méthyle et 9 % du dérivé chloré correspondant.

Après traitement classique du milieu réactionnel (lavages aqueux, extraction au chlorure de méthylène, séchage, filtration et concentration) le brut réactionnel est distillé (45°, 17 mbar) pour conduire au S(-) bromopropionate de méthyle.

L'invention de configuration s'est produite avec un rendement de 98 % comme confirmé par une analyse par CPG en colonne chirale ( D/(L + D) du bromopropionate = 4,3 % )

Rendement énantiomérique : 98%.

EXEMPLE 3

Cet exemple concerne la préparation du bromo-2 octanol.

On additionne en 1 heure 13,02 g (0,1 mole) d'octanol-2 au chlorure de thionyle (13,09 g; 0,11 mole) maintenu à 10°C. Après 25 mn sous agitation et balayage d'argon pour chasser l'HCl libéré, on additionne 8,8 g (0,11 mole) d'HBr gaz en 1 heure à 10°C.

On ajoute alors la triéthylamine (1 g; 0,01 mole) à 5°C puis on laisse revenir le milieu réactionnel à température ambiante. On observe une exothermie (35°C) et un fort dégagement gazeux. On laisse une heure sous agitation à 35°C.

Après traitement classique, on récupère 12,44 g de brut réactionnel titrant 75% en produit bromé attendu (Rendement = 48%).

EXEMPLE 4

Cet exemple concerne la préparation du bromure d'allyle.

A 13,09 g (0,11 mole) de chlorure de thionyle refroidis à 10°C, on additionne en 45 mn l'alcool allylique (5,80 g; 0,1 mole).

Après 45 mn d'agitation on ajoute 8,8 g d'HBr gaz au milieu réactionnel maintenu à une température de 10°C (durée de l'addition : 1 heure 10).

Le milieu est agité pendant 1 heure puis purgé à l'argon pour chasser l'HCl libéré. Il apparaît alors que le bromure d'allyle s'est formé spontanément.

EXEMPLE 5

Cet exemple décrit une autre synthèse du S(-)bromopropionate de méthyle.

Dans un tétracol de 50 ml muni d'un réfrigérant ascendant surmonté d'une tête à hydrogéner relié à un bulleur et à un piège à HCl, d'un thermomètre et d'une arrivée de gaz reliée à une bouteille d'HBr, on charge 10 g (54 mmoles) de chlorosulfite de D-Lactate de méthyle (titre du lactate en énantiomère D/(L + D) : 96,7%).

On introduit alors 5,6 g d'HBr gazeux (69 mmoles) en 1 heure 10 et à 30°C dans le mélange réactionnel, puis l'on chauffe le mélange réactionnel à 100°C pendant 6 h 05.

Le dosage du brut réactionnel en chromatographe phase gazeuse montre que le mélange contient 6,75 g de bromopropionate soit un rendement de 75%.

On reprend ensuite le mélange réactionnel par 30 ml de $CH_2Cl_2$, on lave par 6 x 30 ml d'eau, on sèche sur $Na_2SO_4$ et l'on évapore le solvant.

L'analyse par CPV chirale du bromopropionate obtenu montre qu'il contient 93,9% de l'énantiomère S(-) soit un rendement énantiomérique de 94%.

EXEMPLE 6

Cet exemple concerne la synthèse du S(-)bromopropionate de méthyle à partir du chlorosulfite de D-Lactate de méthyle.

Dans le même tétracol de 50 ml que l'exemple 5, on charge 10,01 g de chlorosulfite de D-Lactate de méthyle (0,054 mole), 43 µl de pyridine (0,53 mmole). On introduit ensuite dans le mélange réactionnel 4,9 g de HBr gazeux (0,060 mole) en 1 heure en maintenant la température entre 25 et 30°C. On maintient ensuite le mélange réactionnel 15 mn à température ambiante et l'on chauffe 1 heure à 80°C puis l'on refroidit.

L'analyse CPV du brut réactionnel montre qu'il contient 8,59 g de bromopropionate soit un rendement de 95,6%.

On reprend ensuite le brut réactionnel par 30 ml de $CH_2Cl_2$, on lave à l'eau jusqu'à neutralité, on sèche le produit sous $Na_2SO_4$, et on évapore le solvant.

L'analyse CPV chirale du produit obtenu montre que le bromopropionate obtenu contient 92,4% de l'énantiomère S(-), soit un rendement énantiomérique de 91%.

EXEMPLE 7

Dans un tricol de 25 ml équipé de la même manière que celui de l'exemple 5, on charge 9,97 g de chlorosulfite de D-Lactate de méthyle (53,4 mmole). On introduit alors 4,7 g d'HBr gaz en 50 mn en maintenant la température entre 25 et 30°C. On ajoute ensuite 43 µl de pyridine (0,54 mmole) et l'on porte le mélange réactionnel à 80°C pendant 35 mn.

L'analyse CPV du brut réactionnel montre qu'il contient 8,24 g de bromopropionate soit un rendement de 92,4%.

L'analyse CPV chirale du brut montre que le bromopropionate obtenu contient 94,6% de S (-) bromopropionate soit un rendement énantiomérique de 95%.

## Revendications

**1.** Procédé de préparation d'un bromoformiate, bromosulfite ou bromophosphite dérivé d'un alcool primaire ou secondaire caractérisé en ce qu'il consiste à faire réagir :

- un cnloroformiate, chlorosulfite, chlorophosphite dérivé d'un alcool primaire ou secondaire répondant respectivement aux formules (3), (4), (5) et (6) avec un agent bromant :

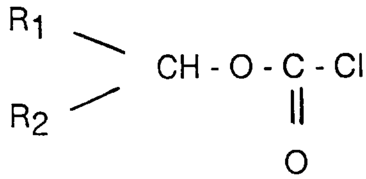
(3)

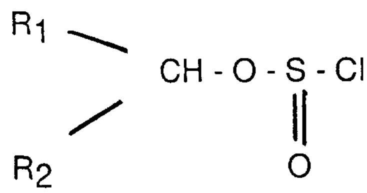
(4)

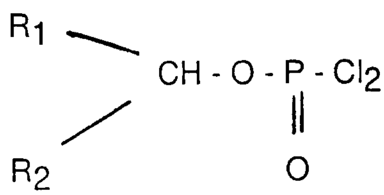
(5)

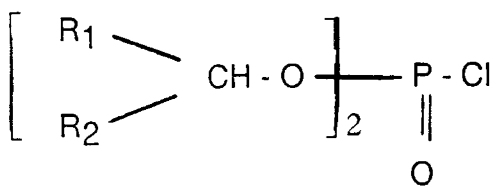
(6)

dans lesquelles :

- $R_1$ et $R_2$ peuvent être identiques ou différents et représentent l'hydrogène, un radical alkyle, alcényle, alcynyle, ces radicaux pouvant être linéaires ou ramifiés, un radical cycloalkyle, alkylcycloalkyle, terpénique -$CO_2R_3$, -$(CH_2)_n$ -$CO_2R_3$, -$COR_3$, -$SOR_3$, -$SO_2R_3$, n étant un nombre entier ; $R_3$ étant l'hydrogène ou un radical alkyle, alcényle, alcynyle, ces radicaux pouvant être linéaires ou ramifiés, cycloalkyle, alkylcycloalkyle, terpénique, aryle notamment aryle substitué,
- au moins un de $R_1$ et $R_2$ pouvant en outre former un hétérocycle comportant en remplacement d'un ou plusieurs atomes de carbone un ou plusieurs hétéroatomes choisis parmi l'oxygène, le soufre ou l'azote.

- ou le phosgène, le chlorure de thionyle ou l'oxychlorure de phosphore avec un agent bromant, puis mis en contact du mélange réactionnel ou du produit de la réaction ainsi obtenu avec un alcool primaire ou secondaire de formule :

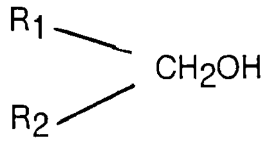
(1)

dans laquelle :

- $R_1$ et $R_2$ peuvent être identiques ou différents et représentent l'hydrogène, un radical alkyle, alcényle, alcynyle, ces radicaux pouvant être linéaires ou ramifiés, un radical cycloalkyle, alkylcycloalkyle, terpénique -$CO_2R_3$, $(CH_2)_n$ -$CO_2R_3$, -$COR_3$, -$SOR_3$, -$SO_2R_3$, n étant un nombre entier ; $R_3$ étant l'hydrogène ou un radical alkyle, alcényle, alcynyle, ces radicaux pouvant être linéaires ou ramifiés, cycloalkyle, alkylcycloalkyle, terpénique, aryle notamment aryle substitué,

- au moins un de $R_1$ et $R_2$ pouvant en outre former un hétérocycle comportant en remplacement d'un ou plusieurs atomes de carbone un ou plusieurs hétéroatomes choisis parmi l'oxygène, le soufre ou l'azote.

dans ledit procédé, l'agent bromant utilisé étant un bromure acide ou un bromure d'alkylammonium.

**2.** Procédé selon la revendication 1 caractérisé en ce que le chloroformiate, le chlorosulfite ou le chlorophosphite est préparé par réaction du phosgène, chlorure de thionyle ou oxychlorure de phosphore avec un alcool primaire ou secondaire, puis le milieu réactionnel ou le produit obtenu à l'issue de l'étape précédente est mis en contact avec un agent bromant.

**3.** Procédé selon l'une des revendications précédentes, caractérisé en ce qu'on utilise des alcools saturés primaires ou secondaires, des alcools éthyléniques primaires ou secondaires, des acides lactiques ou lactates ou les dérivés précités correspondants.

**4.** Procédé selon l'une des revendications 1 et 2, caractérisé en ce qu'on utilise des produits dans laquelle au moins un de $R_1$ et $R_2$ est un hétérocycle du type furanne ou pyranne.

**5.** Procédé selon l'une des revendications 1 à 4 caractérisé en ce que l'on utilise comme agent bromant l'acide bromhydrique, les bromures de métaux alcalins, les bromhydrates d'amines tertiaires aliphatiques ou aromatiques.

**6.** Procédé selon l'une des revendications 1 à 5, caractérisé en ce qu'on utilise comme agent bromant un bromure de tétraburylammonium.

**7.** Procédé de préparation d'un dérivé bromé répondant à la formule (2) :

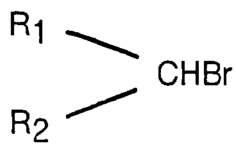

(2)

dans laquelle :

- $R_1$ et $R_2$ peuvent être identiques ou différents et représentent l'hydrogène, un radical alkyle, alcényle, alcynyle, ces radicaux pouvant être linéaires ou ramifiés, un radical cycloalkyle, alkylcycloalkyle, terpénique $-CO_2R_3$, $-(CH_2)_n-CO_2R_3$, $-COR_3$, $-SOR_3$, $-SO_2R_3$, n étant un nombre entier ; $R_3$ étant l'hydrogène ou un radical alkyle, alcényle, alcynyle, ces radicaux pouvant être linéaires ou ramifiés, cycloalkyle, alkylcycloalkyle, terpénique, aryle notamment aryle substitué,
- au moins un de $R_1$ et $R_2$ pouvant en outre former un hétérocycle comportant en remplacement d'un ou plusieurs atomes de carbone un ou plusieurs hétéroatomes choisis parmi l'oxygène, le soufre ou l'azote,

caractérisé en ce qu'il comprend au moins une étape de préparation de bromoformiate, bromosulfite ou bromophosphite selon l'une des revendications 1 à 6.

**8.** Procédé selon la revendication 7 caractérisé en ce qu'il comprend une étape supplémentaire dans laquelle on chauffe le milieu réactionnel ou le produit obtenu à l'issue de la ou des étapes précédentes.

**9.** Procédé selon l'une des revendications 7 et 8 caractérisé en ce qu'il comprend une étape supplémentaire dans laquelle on met en contact avec un composé comprenant un azote trisubstitué, le milieu réactionnel ou le produit issu de la ou des étapes précédentes.

**10.** Procédé selon la revendication 7 caractérisé en ce qu'il consiste à mettre en contact le chloroformiate, le chlorosulfite ou le chlorophosphite de départ, ou issu de la réaction avec l'alcool, avec un composé comprenant un azote trisubstitué, préalablement à l'étape précitée de réaction avec l'agent bromant.

**11.** Procédé selon la revendication 7, caractérisé en ce qu'il consiste à mettre en contact le chloroformiate, le chlorosulfite ou le chlorophosphite de départ ou issu de la réaction avec l'alcool avec un composé comprenant un azote trisubstitué, simultanément avec la réaction avec l'agent bromant.

**12.** Procédé selon la revendication 10 et 11 caractérisé en ce qu'il comprend une étape supplémentaire dans laquelle on chauffe le milieu réactionnel ou le produit issu de la ou des étapes précédentes.

**13.** Procédé selon l'une des revendications 9 et 11 caractérisé en ce que le composé comprenant un azote trisubstitué est choisi dans le groupe comprenant les amines tertiaires aliphatiques et aromatiques, la pyridine et ses dérivés, les amides et formamides, les dérivés de l'urée.

**14.** Procédé selon l'une des revendications précédentes caractérisé en ce qu'au moins une des étapes précitées est effectuée en masse.

**15.** Procédé selon l'une des revendications précédentes caractérisé en ce qu'au moins une des étapes précitées est effectuée en présence d'un solvant.

**Claims**

**1.** A method for the preparation of a bromoformate, bromosulphite or bromophosphite derivative of a primary or secondary alcohol, characterised in that it consists in reacting:

- a chloroformate, chlorosulphite or chlorophosphite derivative of a primary or secondary alcohol corresponding respectively to formulae (3), (4), (5) and (6) with a brominating agent:

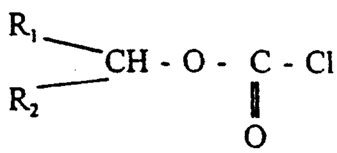

(3)

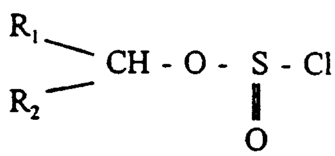

(4)

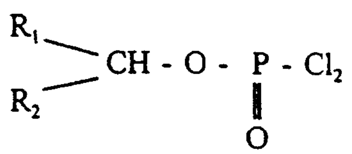

(5)

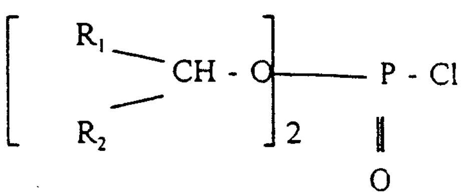

(6)

wherein:

- $R_1$ and $R_2$ can be identical or different and represent hydrogen, an alkyl, alkenyl or alkynyl radical, these radicals being able to be linear or branched, a cycloalkyl, alkylcycloalkyl, terpene $-CO_2R_3$, $-(CH_2)_n-CO_2R_3$, $-COR_3$, $-SOR_3$, or $-SO_2R_3$ radical, n being a whole number; $R_3$ being hydrogen or an alkyl, alkenyl or alkynyl radical, these radicals being able to be linear or branched or a cycloalkyl, alkylcycloalkyl, terpene or aryl, particularly a substituted aryl, radical,
- at least one of $R_1$ and $R_2$ additionally being able to form a heterocyclic compound comprising one or more heteroatoms selected from oxygen, sulphur and nitrogen, instead of one or more carbon atoms.

- or phosgene, thionyl chloride or phosphorus oxychloride with a brominating agent, then putting the reaction mixture or product from the reaction thus obtained into contact with a primary or secondary alcohol of the formula:

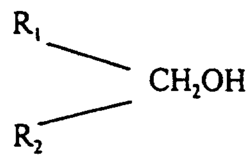
(1)

wherein:

- $R_1$ and $R_2$ can be identical or different and represent hydrogen, an alkyl, alkenyl or alkynyl radical, these radicals being able to be linear or branched, a cycloalkyl, alkylcycloalkyl, terpene $-CO_2R_3$, $-(CH_2)_n-CO_2R_3$, $-COR_3$, $-SOR_3$, $-SO_2R_3$ radical, n being a whole number; $R_3$ being hydrogen or an alkyl, alkenyl or alkynyl radical, these radicals being able to be linear or branched, or a cycloalkyl, alkylcycloalkyl, terpene, or aryl, particularly substituted aryl, radical,
- at least one of $R_1$ and $R_2$ also being capable of forming a heterocyctic compound comprising one or more heteroatoms selected from oxygen, sulphur or nitrogen instead of one or more carbon atoms.

in which method the brominating agent used is an acid bromide or an alkylammonium bromide.

**2.** A method according to Claim 1, characterised in that the chloroformate, chlorosulphite or chlorophosphite is prepared by reacting phosgene, thionyl chloride or phosphorus oxychloride with a primary or secondary alcohol, then the reaction medium or the product obtained at the end of the previous step is put into contact with a brominating agent.

**3.** A method according to one of the preceding claims, characterised by the use of primary or secondary saturated alcohols, primary or secondary ethylene alcohols, lactic acids or lactates, or the corresponding afore-mentioned derivatives.

**4.** A method according to either Claim 1 or Claim 2, characterised in that use is made of products in which at least one of $R_1$ and $R_2$ is a heterocyclic compound of the furan or pyran type.

**5.** A method according to one of Claims 1 to 4, characterised in that hydrobromic acid, alkali metal bromides or hydrobromides of aliphatic or aromatic tertiary amines are used as the brominating agent.

**6.** A method according to one of Claims 1 to 5, characterised in that a tetrabutylammonium bromide is used as the brominating agent.

**7.** A method for the preparation of a brominated derivative corresponding to formula (2):

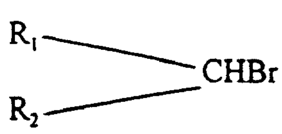
(2)

wherein:

- $R_1$ and $R_2$ can be identical or different and represent hydrogen, an alkyl, alkenyl or alkynyl radical, these radicals being able to be linear or branched, or a cycloalkyl, alkylcycloalkyl, terpene, $-CO_2R_3$, $-(CH_2)_n-CO_2R_3$, $-COR_3$, $-SOR_3$, $-SO_2R_3$ radical, n being a whole number; $R_3$ being hydrogen or an alkyl, alkenyl, akynyl radical, these radicals being able to be linear or branched, cycloalkyl, alkylcycloalkyl, terpene, or aryl, particularly substituted aryl, radicals,
- at least one of $R_1$ and $R_2$ additionally being able to form a heterocyclic compound comprising one or more heteroatoms selected from oxygen, sulphur or nitrogen, instead of one or more carbon atoms,

characterised in that it comprises at least one step for the preparation of bromoformate, bromosulphite or bromophosphite according to one of Claims 1 to 6.

8. A method according to Claim 7, characterised in that it comprises an additional step in which the reaction medium or the product obtained at the end of the preceding step(s) is heated.

9. A method according to either Claim 7 or Claim 8, characterised in that it comprises an additional step in which the reaction medium or the product resulting from the preceding step(s) is put into contact with a compound comprising a tri-substituted nitrogen.

10. A method according to Claim 7, characterised in that it consists putting the initial chloroformate, chlorosulphate or chlorophosphite, or the chloroformate, chlorosulphite or chlorophosphite resulting from the reaction with alcohol into contact with a compound comprising tri-substituted nitrogen, before said stage of reaction with the brominating agent.

11. A method according to Claim 7, characterised in that it cosists in putting the initial chloroformate, chlorosulphite or chlorophosphite, or the chloroformate, chlorosulphite or chlorophosphite resulting from the reaction with alcohol into contact with a compound comprising a tri-substituted nitrogen, simultanously with the reaction with the brominating agent.

12. A method according to Claims 10 and 11, characterised in that it comprises an additional step in which the reaction medium or the product resulting from the previous step(s) is heated.

13. A method according to either Claim 9 or Claim 11, characterised in that the compound comprising a tri-substituted nitrogen is selected from the group comprising aliphatic and aromatic tertiary amines, pyridine and its derivatives, amides and formamides and urea derivatives.

14. A method according to one of the preceding claims, characterised in that at least one of the afore-mentioned steps is carried out in mass.

15. A method according to one of the preceding claims, characterised in that at least one of the afore-mentioned steps is carried out in the presence of a solvent.

**Patentansprüche**

1. Verfahren zur Herstellung eines Bromformiats, Bromsulfids oder Bromphosphits, das sich von einem primären oder sekundären Alkohol ableitet, dadurch gekennzeichnet, daß man:

- ein Chlorformiat-, Chlorsulfid- oder Chlorphosphitderivat eines primären oder sekundären Alkohols, das den Formel (3), (4), (5) bzw. (6) entspricht, mit einem Bromierungsreagenz reagieren läßt

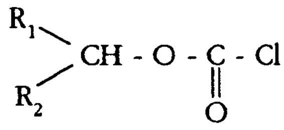

(3)

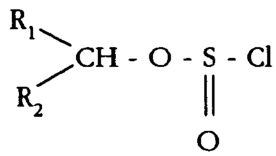

(4)

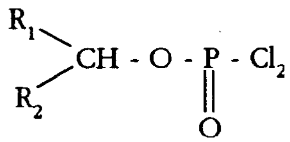

(5)

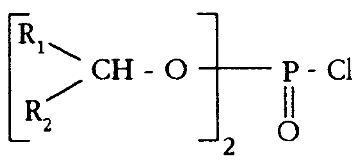

(6)

wobei:

- $R_1$ und $R_2$, identisch oder verschieden, Wasserstoff, einen Alkyl-, Alkenyl- oder Alkinylrest, wobei diese Reste linear oder verzweigt sein können, einen Cycloalkylrest, einen Alkylcycloalkylrest, einen Terpenrest $-CO_2R_3$, $-(CH_2)_n-CO_2R_3$, $-COR_3$, $-SOR_3$, $-SO_2R_3$, wobei n eine ganze Zahl ist, darstellen; $R_3$ Wasserstoff oder ein Alkyl-, ein Alkenyl- oder Alkinylrest, wobei diese Reste linear oder verzweigt sein können, ein Cycloalkylrest, ein Alkylcycloalkylrest, ein Terpenrest, ein Arylrest, insbesondere ein substituierter Arylrest, ist,
- wobei mindestens einer der Reste $R_1$ und $R_2$ außerdem einen Heterocyclus bilden kann, in dem ein oder mehrere Kohlenstoffatome durch ein oder mehrere Heteroatome ersetzt sind, die ausgewählt sind aus Sauerstoff, Schwefel oder Stickstoff;

• oder man Phosgen, Thinylchlorid oder Phosphoroxychlorid mit einem Bromierungsreagenz reagieren läßt, man anschließend die so erhaltene Reaktionsmischung oder das so erhaltene Reaktionsprodukt mit einem primären oder sekundären Alkohol der Formel

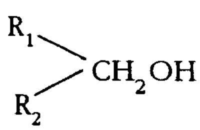

(1)

in Kontakt bringt, wobei:

- $R_1$ und $R_2$, identisch oder verschieden, Wasserstoff, einen Alkyl-, Alkenyl- oder Alkinylrest, wobei diese Reste linear oder verzweigt sein können, einen Cycloalkylrest, einen Alkylcycloalkylrest, einen Terpenrest $-CO_2R_3$, $-(CH_2)_n-CO_2R_3$, $-COR_3$, $-SOR_3$, $-SO_2R_3$, wobei n eine ganze Zahl ist, darstellen; $R_3$ Wasserstoff oder ein Alkyl-, ein Alkenyl- oder Alkinylrest, wobei diese Reste linear oder verzweigt sein können, ein Cycloalkylrest, ein Alkylcycloalkylrest, ein Terpenrest, ein Arylrest, insbesondere ein substituierter Arylrest, ist,
- wobei mindestens einer der Reste $R_1$ und $R_2$ außerdem einen Heterocyclus bilden kann, in dem ein oder

mehrere Kohlenstoffatome durch ein oder mehrere Heteroatome ersetzt sind, die ausgewählt sind aus Sauerstoff, Schwefel oder Stickstoff;

wobei in diesem Verfahren das verwendete Bromierungsreagenz ein saures Bromid oder ein Akylammoniumbromid ist.

**2.** Verfahren nach Anspruch 1, dadurch gekennzeichnet, daß das Chlorformiat, Chlorsulfid oder Chlorphosphit durch Reaktion von Phosgen, Thionylchlorid oder Phosphoroxychlorid mit einem primären oder sekundären Alkohol hergestellt wird, dann das am Ende des vorhergehenden Verfahrensschrittes erhaltene Reaktionsmilieu oder Produkt mit einem Bromierungsreagenz in Kontakt gebracht wird.

**3.** Verfahren nach einem der vorhergehenden Ansprüche, dadurch gekennzeichnet, daß man primäre oder sekundäre gesättigte Alkohole, primäre oder sekundäre ethylenische Alkohole, Milchsäuren oder lactate oder die entsprechenden, zuvor genannten Derivate verwendet.

**4.** Verfahren nach Anspruch 1 oder 2, dadurch gekennzeichnet, daß man Produkte verwendet, in denen mindestens einer der beiden Reste $R_1$ und $R_2$ ein Heterocyclus vom Furan- oder Pyrantyp ist.

**5.** Verfahren nach einem der Ansprüche 1 bis 4, dadurch gekennzeichnet, daß man als Bromierungsreagenz Bromwasserstoffsäure, Alkalimetallbromide oder Bromhydrate von tertiären aliphatischen oder aromatischen Antinen verwendet.

**6.** Verfahren nach einem der Ansprüche 1 bis 5, dadurch gekennzeichnet, daß man als Bromierungsreagenz ein Tetrabutylammoniumbromid verwendet.

**7.** Verfahren zur Herstellung eines Bromderivats, das der Formel (2) entspricht:

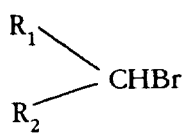

(2)

in der:

- $R_1$ und $R_2$, identisch oder verschieden, Wasserstoff, einen Alkyl-, Alkenyl- oder Alkinylrest, wobei diese Reste linear oder verzweigt sein können, einen Cycloalkylrest, einen Alkylcycloalkylrest, einen Terpenrest $-CO_2R_3$, $-(CH_2)_n-CO_2R_3$, $-COR_3$, $-SOR_3$, $-SO_2R_3$, wobei n eine ganze Zahl ist, darstellen; $R_3$ Wasserstoff oder ein Alkyl-, Alkenyl- oder Alkinylrest, wobei diese Reste linear oder verzweigt sein können, ein Cycloalkylrest, ein Alkylcycloalkylrest, ein Terpenrest, ein Arylrest, insbesondere ein substituierter Arylrest, ist,
- wobei mindestens einer der Reste $R_1$ und $R_2$ außerdem einen Heterocyclus bilden kann, in dem ein oder mehrere Kohlenstoffatome durch ein oder mehrere Heteroatome ersetzt sind, die ausgewählt sind aus Sauerstoff, Schwefel oder Stickstoff;

dadurch gekennzeichnet, daß es mindestens einen Verfahrensschritt zur Herstellung von Bromformiat, Bromsulfid oder Bromphosphit nach einem der Ansprüche 1 bis 6 umfaßt.

**8.** Verfahren nach Anspruch 7, dadurch gekennzeichnet, daß es einen weiteren Verfahrensschritt umfaßt, bei dem man das am Ende des oder der vorhergehenden Verfahrensschritte erhaltene Reaktionsmilieu oder Produkt erhitzt.

**9.** Verfahren nach Anspruch 7 oder 8, dadurch gekennzeichnet, daß es einen zusätzlichen Verfahrensschritt umfaßt, bei dem man das am Ende des oder der vorhergehenden Verfahrensschritte erhaltene Reaktionsmilieu oder Produkt mit einer Verbindung in Kontakt bringt, die einen dreifach substituierten Stickstoff umfaßt.

**10.** Verfahren nach Anspruch 7, dadurch gekennzeichnet, daß man das Ausgangschlorformiat, -chlorsulfid oder -chlorphosphit oder das aus der Reaktion mit dem Alkohol stammende Chlorformiat, Chlorsulfid oder Chlorphosphlt vor dem zuvor genannten Verfahrensschritt, der die Reaktion mit dem Bromierungsreagenz umfaßt, mit einer Verbindung in Kontakt bringt, die einen dreifach substituierten Stickstoff umfaßt.

11. Verfahren nach Anspruch 7, dadurch gekennzeichnet, daß man das Ausgangschlorformiat, -chlorsulfid oder -chlorphosphit oder das aus der Reaktion mit dem Alkohol stammende Chlorformiat, Chlorsulfid oder Chlorphosphit gleichzeitig mit der Reaktion mit dem Bromierungsreagenz mit einer Verbindung in Kontakt bringt, die einen dreifach substituierten Stickstoff umfaßt.

12. Verfahren nach Anspruch 10 oder 11, dadurch gekennzeichnet, daß es einen zusätzlichen Verfahrensschritt umfaßt, bei dem man das am Ende des oder der vorhergehenden Verfahrensschritte erhaltene Reaktionsmilieu oder Produkt erhitzt.

13. Verfahren nach einem der Ansprüche 9 und 11, dadurch gekennzeichnet, daß die Verbindung, die einen dreifach substituierten Stickstoff umfaßt, ausgewählt ist aus der Gruppe von tertiären aliphatischen und aromatischen Aminen, Pyridin und seinen Derivaten, Amiden und Formamiden sowie Derivaten des Hamstoffes.

14. Verfahren nach einem der vorhergehenden Ansprüche, dadurch gekennzeichnet, daß mindestens einer der zuvor genannten Verfahrensschritte in Masse durchgeführt wird.

15. Verfahren nach einem der vorhergehenden Ansprüche, dadurch gekennzeichnet, daß mindestens einer der zuvor genannten Verfahrensschritte in Gegenwart eines Lösemittels durchgeführt wird.